**Europäisches Patentamt**

**European Patent Office**

**Office européen des brevets**

(19)

(11) Veröffentlichungsnummer: **0 081 791**
A1

(12) # EUROPÄISCHE PATENTANMELDUNG

(21) Anmeldenummer: **82111287.7**

(22) Anmeldetag: **06.12.82**

(51) Int. Cl.³: **A 61 L 9/12**, B 65 D 75/62

(30) Priorität: **14.12.81 DE 3149508**

(43) Veröffentlichungstag der Anmeldung: **22.06.83**
**Patentblatt 83/25**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf 1 (DE)**

(72) Erfinder: **Künzel, Werner, Eichfeldstrasse 65, D-4018 Langenfeld (DE)**

(54) **Duftbeutel und Verfahren zum Herstellen.**

(57) Der Duftbeutel (1) besitzt zwei an den Rändern (4, 5, 6) miteinander verbundene und den Duftstoff (26) umschließende, aromadichte Folienflächen (7, 8). Die eine der Folienflächen (7) soll Perforationen (9) zum Öffnen des Beutels aufweisen.

Die eine Beutelseite (7) wird aus einer aromadurchlässigen Innenschicht (2) und einer aromadichten Außenschicht (3) zusammengesetzt, wobei nur die letztere beim Öffnen abzuziehen ist.

Henkelstr. 67
4000 Düsseldorf, den 11. 12. 1981

HENKEL KGaA
ZR-FE/Patente
Bor/C

— 1 —

0081791

P a t e n t a n m e l d u n g
D 6462 EP

"Duftbeutel und Verfahren zum Herstellen"

Die Erfindung betrifft einen Duftbeutel mit zwei an den Rändern miteinander verbundenen und den Duftbeutel umschließenden, aromadichten Folienflächen, von denen die eine bei Anwendung wenigstens zum Teil, insbesondere durch Aufreißen längs einer Perforation, zu entfernen ist. Sie betrifft ferner ein Verfahren zum Herstellen des Duftbeutels.

Es gibt auf dem Markt eine Vielzahl von Duftspende-Systemen, die an den verschiedensten Orten bzw. Innenräumen von Geräten zwecks Überlagerung anderer Gerüche eingesetzt werden. Die meisten dieser Systeme werden vor ihrer Anwendung zum Vermeiden unnötiger oder unerwünschter Duftstoffabgaben während des Transports oder der Lagerung in einer aromadichten Umverpakkung, z.B. in einem Beutel, in Schachteln, Dosen, Flaschen und ähnlichem, geschützt aufbewahrt. Erst unmittelbar vor Anwendung des Duftspenders wird die Außenverpackung entfernt.

Die meisten handelsüblichen Duftspende-Systeme lassen auch bei offener bzw. teilweise offener Verpackung ein Entweichen des Dufts in die Atmosphäre zu. Teilweise, z.B. bei WC-Duftspendern, wird eine verstärkte Duftstoffabgabe durch Einwirkung von Wasser auf das als Festkörper oder -masse vorliegende Duftprodukt bewirkt. Es gibt auch Duftspender mit als Flüssigkeit in Form eines Konzentrats eingesetztem Produkt. Hierbei wird natürlich eine flüssigkeitsdichte Verpackung benötigt, auch kann die Duftflüssigkeit mit Hilfe eines Trägermaterials,

Patentanmeldung

HENKEL KGaA
ZR-FE/Patente

D 6462 EP                2

z.B. Fließmaterial oder Schwamm, in der Verpackung flächig verteilt und festgehalten werden. Bei allen bekannten Systemen besteht die Gefahr des Hautkontakts mit dem Produkt; außerdem kann das Produkt, z.B. wenn es durch Wasserkontakt, wie bei WC-Duftsteinen, abgetragen wird, zur Umweltbelastung beitragen.

Der Erfindung liegt die Aufgabe zugrunde, einen Duftstoffbeutel zu schaffen, der sowohl einen guten Aromaschutz beim Transport und Lagern gewährleistet als auch nach einfacher Manipulation als Duftspender ohne die Gefahr eines Hautkontakts oder der Umweltbelastung einzusetzen ist. Die erfindungsgemäße Lösung besteht für den Duftbeutel darin, daß die Folienfläche auf der einen Beutelseite aus einer aus einer aromadurchlässigen Innenschicht und einer aromadichten Außenschicht bestehenden Doppelschicht zusammengesetzt ist. Vorzugsweise werden die Schichten der Doppelschicht nur an den Rändern untereinander und mit den Ränden der anderen Beutelseite verbunden, insbesondere versiegelt bzw. verschweißt.

Durch die Erfindung wird demgemäß erreicht, daß der Duftbeutel gleichzeitig in Form seiner konstruktiven Ausführung aromageschützt, transport- und lagergeeignet und durch geringfügige, unten beschriebene Manipulation als Duftspender einsetzbar ist. Weiterhin wird der erfindungsgemäße Duftbeutel in seiner Folienzusammensetzung so aufgebaut, daß während der Duftstoffabgabe jeglicher Haut- und/oder Wasserkontakt zu vermeiden ist, d.h. das System bleibt "geschlossen" mit der weiteren Folge, daß der Aggregatzustand des duftabgebenden Produkts frei zu wählen ist und je nach Anforderung und Einsatzzweck flüssig, pastenartig oder fest sein kann.

0081791

HENKEL KGaA
ZR-FE/Patente

D 6462 EP                3

Gemäß weiterer Erfindung wird der Duftbeutel hergestellt, indem die drei Folienschichten zugleich von Rolle abgezogen, mit Hilfe von Längs- und Querschweißwerkzeugen zu einem Beutel geformt, mit dem Duftstoff gefüllt, verschlossen und mit einem Trennmesser von dem laufenden Material gelöst werden. Vorzugsweise wird dabei ein sogenannter Vier-Seiten-Siegelrandbeutel hergestellt, der auf entsprechenden Beutel-Form-Füll- und -Verschließmaschinen produktionsmäßig herzustellen ist.

Das Rückenmaterial des erfindungsgemäßen Beutels kann aus einer aromadichten Verbundfolie, z.B. aus einer Polyäthylen/Aluminium/Polyester-Verbundfolie, bestehen. Dieses Verbundmaterial wird bei der Herstellung des Beutels von der Rolle abgezogen. Die Vorderseite des Beutels, die später auch aromadurchlässig sein soll, besteht erfindungsgemäß aus zwei Folientypen, die beim Herstellen des Beutels in der Regel von zwei getrennten Rollen abzuziehen sind. Die Reihenfolge der Folien wird dabei erfindungsgemäß so gewählt, daß die an der Beutelinnenseite liegende, also zum Produkt hingewandte Folie, aromadurchlässig ist und z.B. aus Polyäthylen besteht. Als aromadichte Folie kann beispielsweise eine Aluminiumfolie verwendet werden. Die beiden Folien können flächig, beispielsweise mit Hilfe eines Peel-Lacks oder durch Schweißen bzw. Siegeln nur an den vier Kanten miteinander verbunden werden.

Vor Anwendung des erfindungsgemäßen Duftbeutels ist die aromadichte Schicht der Doppelschichtseite wenigstens zum Teil abzuziehen. Zur Erleichterung dieses Aufreißens können in der aromadichten Folie der Doppelschichtseite vorzugsweise unmittelbar nach dem Rollenabzug - also vor dem Zusammenlaufen mit

. . .

: : : : : : : : : : : : : : 0081791

D 6462 EP                          4

der aromadurchlässigen Schicht - in Längsrichtung, d.h. in der Rollenabzugsrichtung, verlaufende Perforationslinien neben den am Beutelrand vorgesehenen Längsverbindungsnähten gebildet werden. Um das Abziehen der am Rand perforierten Außenfolie zu erleichtern, kann die Folie mit einem weiteren Stanzwerkzeug, vorzugsweise ebenfalls unmittelbar nach dem Rollenabzug, quergeschlitzt, d.h. in Querrichtung zur Abzugsrichtung unterhalb einer beim Fertigkonfektionieren quer zu den Längsnähten herzustellenden Quernaht mit einem Querschlitz versehen werden. Bei Anwendung kann der Beutel dann im oberen Bereich leicht so abgeknickt werden, daß die sich dabei abhebende Lasche am Ende leicht zu fassen und abziehen ist. Die Flächen der Perforationslinien und der Schlitzung nehmen relativ zur Gesamtfläche der Folie so eine geringe Fläche ein, daß der Duftaustritt aus diesem Bereich vernachlässigbar ist.

Die Schweißverbindung einer aus Aluminium bestehenden Abdeckfolie im Vier-Seiten-Naht-Bereich wird verbessert, wenn die Folie vor dem Schweißen mit einem Siegellack oder aber z.B. mit Polyäthylen beschichtet wird. Nach Abziehen der aromadichten Folie längs der Perforation verbleiben Reste dieser Folie an den Verbindungsnähten des Beutels.

In einem weiteren Ausführungsbeispiel kann die aromadichte Außenfolie vollflächig, z.B. mittels eines Peel-Lacks, mit der aromadurchlässigen Innenfolie verbunden werden. Die aromadichte Folie ist dann vollflächig von der Innenfolie abzuziehen. Auch hierbei ist eine Abzugslasche, z.B. im Eckenbereich des Beutels, zum Ergreifen der Folie bei Beginn des Abziehens sehr vorteilhaft.

D 6462 EP                    5

In vielen Anwendungsfällen, z.B. in der Funktion als Duftspender in Geschirrspülmaschinen, kann es erwünscht sein, den
Beutel um ein bestimmtes Maß zu verlängern, so daß der Beutel
nach der eigentlichen produkteinschließenden oberen Quernaht
eine zweite Quernaht und zwischen den Quernähten symmetrisch
oder asymmetrisch ein, beispielsweise gestanztes, Aufhängeloch besitzen kann. In einem anderen Ausführungsbeispiel wird
zweckmäßig unterhalb der Querschlitzung der äußeren, abzuziehenden, aromadichten Folie ein Loch oder ein kleiner Schlitz
so eingestanzt, daß nach teilweisem Abziehen dieser Folie
der Beutel an dem Loch bzw. Schlitz des als Aufhängelasche
fungierenden Folienteils aufzuhängen ist.

Patentanmeldung

HENKEL KGaA
ZR-FE/Patente

D 6462 EP                 6

Anhand der schematischen Darstellung von Ausführungsbeispielen werden weitere Einzelheiten der Erfindung erläutert. Es zeigen:

Fig. 1    einen Duftbeutel in der Draufsicht;
Fig. 2    einen Duftbeutel ähnlich Fig. 1 jedoch mit Aufhängeloch;
Fig. 3    einen Schnitt durch den Beutel von Fig. 2;
Fig. 4    einen Beutel mit als Aufhängevorrichtung ausgebildeter Lasche;
Fig. 5    den Beutel von Fig. 4 mit abgezogener Lasche; und
Fig. 6    Verfahrensschritte und Werkzeuge zum Herstellen und Füllen des Duftbeutels.

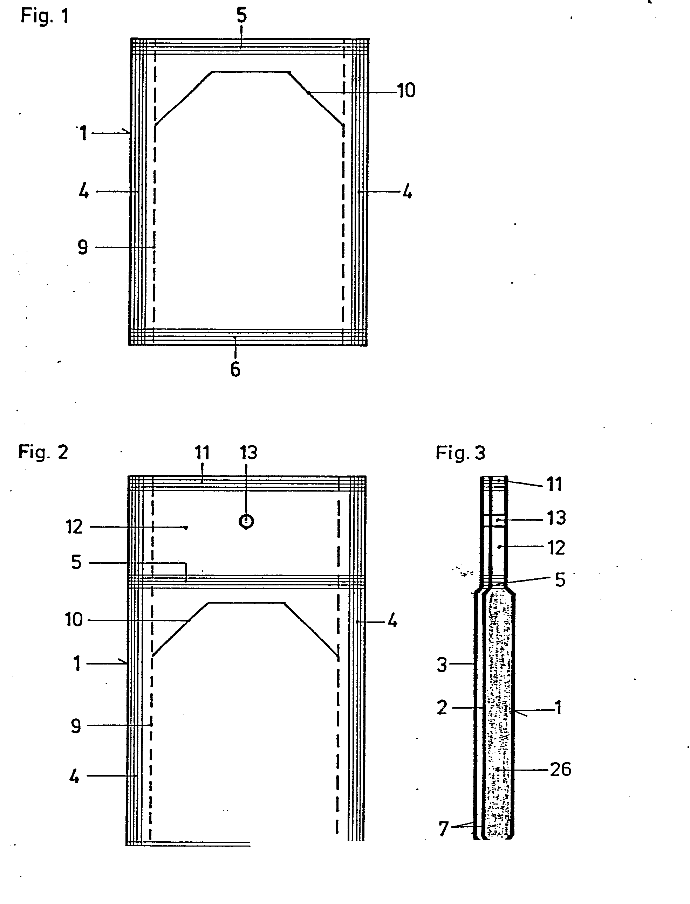

Der in den Fig. 1 bis 6 in verschiedenen Ausführungsbeispielen dargestellte, insgesamt mit 1 bezeichnete Duftbeutel besteht auf der einen Beutelfläche (vergleiche Fig. 3) aus einer aus einer aromadurchlässigen Innenschicht 2 und einer aromadichten Außenschicht 3 bestehenden, im wesentlichen nur an den Längsnähten 4 und Kopf- und Bodennähten 5 und 6 miteinander verbundenen Doppelschicht 7 sowie aus einer aromadichten Rückenschicht 8. Die Ränder der Schichten 2 und 3 der Doppelschicht 7 sind untereinander und mit den Rändern der Rückenschicht 8 versiegelt bzw. verschweißt. Die Außenschicht 3 der Doppelschicht 7 besitzt etwa parallel zu den Schweißnähten an den Längsnähten 4 verlaufende Perforationslinien 9. Außerdem wird in der Außenschicht 3 der Doppelschicht 7 eine die etwa parallel zu den Längsnähten 4 verlaufenden Randperforierungslinien 9 verbindende Querschlitzung 10 vorgesehen. Letztere wird vorzugsweise in Form einer Lasche ausgebildet.

Patentanmeldung

HENKEL KGaA
ZR-FE/Patente

D 6462  EP                    7

Die beiden Schichten 2 und 3 der erfindungsgemäßen Doppelschicht 7 werden häufig nicht flächig, sondern nur am Rande
miteinander verbunden. Zum Verbinden kann der Randstreifen
mit Siegellack oder Polyäthylen beschichtet werden. Falls eine
flächige Verbindung der beiden Schichten 2 und 3 der Doppelschicht 7 gewünscht wird, ist es zweckmäßig, einen lösbaren
Peel-Lack o.ä. zu verwenden. In diesem Falle wird die Außenschicht 3 vorzugsweise mit einer leicht abzuhebenden (nicht
gezeichneten) Abzugslasche in einer Beutelecke versehen.

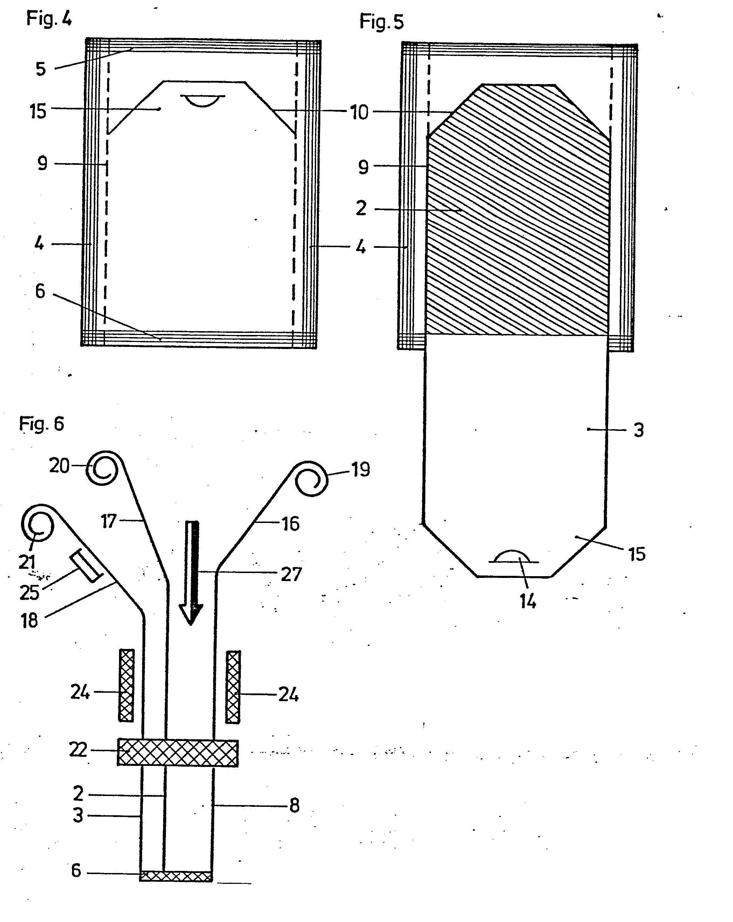

In vielen Fällen ist es wünschenswert, einen Duftbeutel 1 erfindungsgemäßer Art mit einer Aufhängevorrichtung zu versehen.
Eine solche kann dadurch entstehen, daß gemäß Fig. 3 und 4
eine Beutelverlängerung an der Kopfnaht 5 vorgesehen wird,
die ebenfalls eine umlaufende Schweißnaht 11 besitzen kann.
Irgendwo auf der Fläche, z.B. in der Mitte, dieses Erweiterungsbereichs 12 des Beutels 1 kann dann ein Aufhängeloch 13
vorgesehen werden. Stattdessen kann ein Aufhängeloch 14 auch
in der zum Öffnen des Beutels 1 vorgesehenen und einen Teil
der Außenschicht 3 bildenden Lasche 15 vorgesehen werden. Nach
dem Abziehen der Lasche 15 kann der Beutel an dem Aufhängeloch
14 aufgehängt werden.

Zum Herstellen des erfindungsgemäßen Beutels können gemäß
Fig. 6 drei verschiedene Folien 16, 17 und 18 von entsprechenden Folienrollen 19, 20 und 21 abgezogen und den diversen
Werkzeugen sowie der Abfülleinrichtung zugeführt werden. Dabei
wird zunächst in einem Querschweißwerkzeug 22 die Bodennaht 6
des Beutels hergestellt und von der Kopfnaht des vorhergehenden Beutels abgetrennt. Daraufhin werden mit Längsschweißwerkzeugen 24 die Längsnähte 4 des Beutels gebildet. Während des

D 6462 EP                 8

Zuführens der als Außenschicht 3 vorgesehenen Folie 18 zu den Schweißwerkzeugen können mit Hilfe eines Stanzwerkzeugs 25 die Perforationen, Schnitte usw. in die abzuziehende Aussenschicht 3 eingebracht werden. Wenn die Bodennaht 6 und die Randnähte 4 fertiggestellt sind, wird durch das noch offene Kopfende des Beutels Produkt 26 in Pfeilrichtung 27 in den Beutel 1 eingeführt. Abschließend wird die Kopfnaht 5 mit Hilfe des Schweißwerkzeugs 22 und gegebenenfalls eine zusätzliche Quernaht 11 oberhalb eines Erweiterungsbereichs 12 gemäß Fig. 2 und 3 hergestellt.

Patentanmeldung

HENKEL KGaA
ZR-FE/Patente

– 9 –

D 6462   EP

Bezugszeichenliste

| | | |
|---|---|---|
| 1 | = | Beutel |
| 2 | = | Innenfläche |
| 3 | = | Außenfläche |
| 4 | = | Randnähte |
| 5 | = | Kopfnaht |
| 6 | = | Bodennaht |
| 7 | = | Doppelschicht |
| 8 | = | Rückenschicht |
| 9 | = | Perforation |
| 10 | = | Querschlitz |
| 11 | = | Verbindungsnaht |
| 12 | = | Erweiterungsbereich |
| 13, 14 | = | Aufhängeloch |
| 15 | = | Lasche |
| 16 bis 18 | = | Folien |
| 19 bis 21 | = | Rollen |
| 22 | = | Querschweißwerkzeug |
| 23 | = | Messer |
| 24 | = | Längsschweißwerkzeug |
| 25 | = | Stanzwerkzeug |
| 26 | = | Produkt |
| 27 | = | Pfeilrichtung |

Patentanmeldung

HENKEL KGaA
ZR-FE/Patente

D 6462 EP

P a t e n t a n s p r ü c h e

1. Duftbeutel (1) mit zwei an den Rändern (4, 5, 6) untereinander verbundenen und den Duftstoff (26) umschließenden, aromadichten Folienflächen (7, 8), von denen die eine (7) bei Anwendung wenigstens zum Teil, insbesondere durch Aufreißen längs einer Perforation (9), zu entfernen ist, dadurch gekennzeichnet, daß die Folienfläche (7) auf der einen Beutelseite aus einer aus einer aromadurchlässigen Innenschicht (2) und einer aromadichten Außenschicht (3) bestehenden Doppelschicht (7) zusammengesetzt ist.

2. Duftbeutel nach Anspruch 1, dadurch gekennzeichnet, daß die Schichten (2, 3) der Doppelschicht (7) an den Rändern (4, 5, 6) untereinander und mit den Rändern der Rückenschicht (8) verbunden, insbesondere versiegelt bzw. verschweißt, sind.

3. Duftbeutel nach Anspruch 1 oder 2, dadurch gekennzeichnet, daß die Innenschicht (2) und Außenschicht (3) der Doppelschicht (7) vollflächig, insbesondere mit Hilfe eines Peel-Lacks, lösbar miteinander verbunden sind.

4. Doppelschicht nach Anspruch 3, dadurch gekennzeichnet, daß die Außenschicht (3) eine Abzugslasche in einer Beutelecke besitzt.

5. Beutel nach einem oder mehreren der Ansprüche 1 bis 4, dadurch gekennzeichnet, daß die Außenschicht (3) der Doppelschicht (7) etwa parallel zu den Längsnähten (4) verlaufende Perforationslinien (9) besitzt.

Patentanmeldung

HENKEL KGaA
ZR-FE/Patente

D 6462 EP                    *2*

6. Duftbeutel nach einem oder mehreren der Ansprüche 1 bis 5, dadurch gekennzeichnet, daß die Außenschicht (3) der Doppelschicht (7) mindestens einen die Randperforierung (9) verbindenden Querschlitz (10) besitzt.

7. Duftbeutel nach einem oder mehreren der Ansprüche 1 bis 6, dadurch gekennzeichnet, daß die Außenschicht (3) der Doppelschicht (7) in dem zum Verbinden mit der Innenschicht (2) vorgesehenen Bereich mit einem Siegellack oder mit Polyäthylen beschichtet ist.

8. Duftbeutel nach einem oder mehreren der Ansprüche 1 bis 7, dadurch gekennzeichnet, daß die zum Öffnen abzuziehende Außenschicht (3) ein Aufhängeloch (14) enthält.

9. Duftbeutel nach einem oder mehreren der Ansprüche 1 bis 8, dadurch gekennzeichnet, daß oberhalb der die drei Beutelschichten (2, 3, 8) zusammenhaltenden Verbindungslinien an der Kopfnaht (5) ein, insbesondere mit zusätzlichen Verbindungsnähten (11) umgebener, Erweiterungsbereich (12) vorgesehen ist und daß der Erweiterungsbereich (12) ein Aufhängeloch (13) enthält.

10. Verfahren zum Herstellen des Duftbeutels (1) nach einem oder mehreren der Ansprüche 1 bis 9, dadurch gekennzeichnet, daß die drei Beutelschichten (2,3,8) zugleich von getrennten Rollen (19 bis 21) abgezogen, mit Hilfe von Längs- und Querschweiß- oder -siegelwerkzeugen (22, 24) zu einem Beutel geformt, mit dem Duftstoff (26) gefüllt, aromadicht verschlossen und mit einem Trennmesser (23) von dem laufenden Material (16 bis 18) gelöst werden.

Patentanmeldung

HENKEL KGaA
ZR-FE/Patente

D 6462   EP

11. Verfahren nach Anspruch 10, dadurch gekennzeichnet, daß der Beutel (1) vor dem Herstellen der Randverbindungslinien auf der Doppelschichtseite (7) in der Außenfolie (3), insbesondere durch Stanzen, perforiert und/oder mit Einschnitten bzw. Aufhängelöchern versehen wird.

Patentanmeldung
D 6462 EP

**Fig. 1**

5

10

1

4

4

9

6

**Fig. 2**

11    13

12

5

10

4

1

9

4

**Fig. 3**

11

13

12

5

3

2

1

26

7

2/2

Fig. 4

Fig. 5

Fig. 6

Europäisches
Patentamt

**EUROPÄISCHER RECHERCHENBERICHT**

Nummer der Anmeldung

EP 82 11 1287

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| X | GB-A-1 516 845 (D.R.G.(UK) Ltd.) * Insgesamt * | 1-3,7-9 | A 61 L 9/12 B 65 D 75/62 |
| X | US-A-4 145 001 (R.J. WEYENBERG et al) * Spalte 2 Zeile 32 bis Spalte 3 Zeile 32 Patentansprüche * | 1-4,7 | |
| A | | 10 | |
| X | DE-A-2 811 244 (RISDON MANUFACTURING Co.) * Insgesamt * | 1-3,7, 10 | |
| A | DE-A-3 029 476 (B. VAN SCHOOR) * Insgesamt * | 1,4,7 | RECHERCHIERTE SACHGEBIETE (Int. Cl. ³) |
| A | DE-A-2 155 069 (UNIONPACK ABFUELL - UND VERPACKUNGSBETRIEBE) * Seite 8 Zeile 30 bis Seite 11 Zeile 2 Patentansprüche * | 1-5 | A 61 L B 65 D |
| A | US-A-4 220 244 (S. ELMORE) * Insgesamt * | 1,2,4, 5 | |

Der vorliegende Recherchenbericht wurde für alle Patentansprüche erstellt.

| Recherchenort DEN HAAG | Abschlußdatum der Recherche 15-03-1983 | Prüfer FLETCHER A.S. |
|---|---|---|